# EUROPÄISCHE PATENTSCHRIFT

(11) **EP 2 322 925 B1**
(45) Veröffentlichungstag und Bekanntmachung des Hinweises auf die Patenterteilung: **13.02.2013**
(21) Anmeldenummer: 10014501.0
(22) Anmeldetag: 11.11.2010
(51) Int. Cl.: G01N 33/487, B01L 3/00, C12M 3/00, G01N 33/483

(54) **Vorrichtung zur Bestimmung der Impedanz von Zellschichten**
Device for determining the impedance of cell layers
Dispositif de détermination de l'impédance de couches cellulaires

(30) Priorität: 12.11.2009 DE 102009052673
(43) Veröffentlichungstag der Anmeldung: 18.05.2011
(73) Patentinhaber: nanoAnalytics GmbH, 48149 Münster (DE)
(72) Erfinder: Anczykowski, Boris, 48143 Münster (DE); Anczykowski, Bernhard, 48157 Münster (DE); Schäfer, Markus, 48153 Münster (DE)
(74) Vertreter: Hakvoort, Ansgar

(56) Entgegenhaltungen:
- WO-A1-2006/112870
- JP-A- 2006 003 293
- US-A1- 2003 215 940
- US-A1- 2005 221 274

## Beschreibung

Die vorliegende Erfindung betrifft eine Vorrichtung zur Bestimmung der transzellularen Impedanz von auf einer permeablen Membran kultivierten Zellen, ein Verfahren zur Bestimmung der transzellularen Impedanz von auf einer permeablen Membran kultivierten Zellen sowie die Verwendung einer Vorrichtung zur Bestimmung der transzellularen Impedanz von auf einer permeablen Membran kultivierten Zellen.

Der transzellulare elektrische Widerstand einer Zellschicht ist ein Maß für deren Ionenpermeabilität und somit auch ein wesentlicher physikalischer Parameter zur Beurteilung der Barriereeigenschaften kultivierter Zellen. Im Gegensatz zu anderen elektrochemischen Verfahren zur Bestimmung des transzellularen Widerstandes, die entweder mit Gleichspannung oder Wechselspannung mit konstanter Frequenz arbeiten, verwendet die Impedanzspektroskopie eine sinusförmige Wechselspannung, deren Frequenz im Verlaufe der Messung verändert wird. Bei Verwendung kleiner Spannungsamplituden wird so im Gegensatz zu den anderen Messverfahren eine störungsfreie Messung an einer Zellschicht ermöglicht. Diese nicht-invasive Untersuchungsmethode zur Bestimmung elektrochemischer Parameter einer Zellschicht liefert nicht nur den transzellularen Widerstand, es lassen sich durch dieses Verfahren auch oberflächenspezifische Parameter, wie etwa die Kapazität der Zellschicht, sehr genau bestimmen.

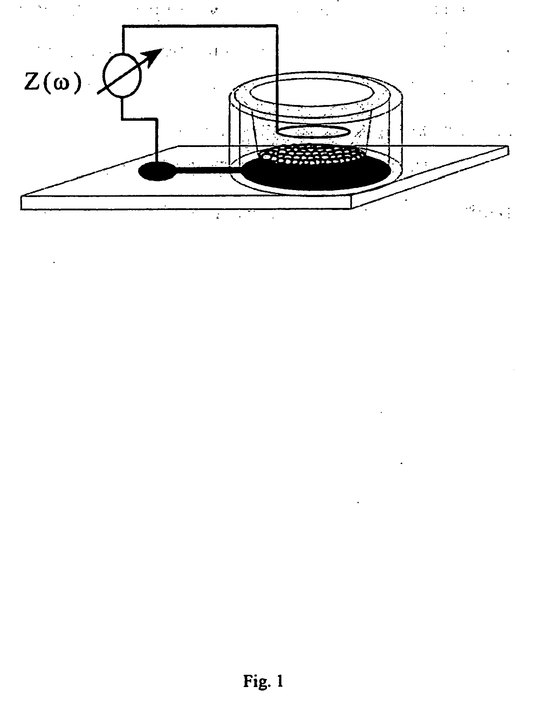

Zur Untersuchung einer Zellschicht mittels Impedanzspektroskopie werden die Zellen üblicherweise auf einhängbaren Membransystemen, welche als Bodenmaterial permeable Membranen aufweisen, zu konfluenten Monolayem kultiviert (sogenannte *"cell culture inserts").* Diese Membransysteme werden dann in Messkammem eingesetzt, deren Boden eine Elektrode, beispielsweise in Form einer Goldbeschichtung, aufweist. Von oben wird die Gegenelektrode in das Membransystem eingeführt. Nachdem sowohl die Messkammer als auch das Membransystem mit einer elektrisch leitfähigen Flüssigkeit, beispielsweise einem Nährmedium, befüllt wurde, lassen sich die Zellschichten impedanzspektroskopisch analysieren. Ein prinzipieller Aufbau eines solchen Messsystems ist in der Figur 1 gezeigt.

Um zugleich mehrere Membransysteme impedanzspektroskopisch analysieren zu können, wurden verschiedene Systeme entwickelt. So beschreibt WO-A-2005/098423 eine Anordnung aus Multiwellplatten umfassend eine erste Anordnung von Behältnissen, wobei in jedes Behältnis eine Silberelektrode hineinragt, sowie eine zweite Anordnung umfassend eine Vielzahl von Membransystemen, wobei die Membransysteme in die Behältnisse eingesetzt werden können. In die Membransysteme ragen von oben die entsprechenden Gegenelektroden hinein. Der Nachteil der in der WO-A-2005/098423 beschriebenen Messordnung besteht unter anderem darin, dass jedes einzelne Behältnis, in welches ein Membransystem eingesetzt wird, eine eigene Silberchloridelektrode aufweist. Dieses bedingt, dass jede einzelne Silberchloridelektrode einzeln kontaktiert werden muss. Darüber hinaus sind Silberchloridelektroden nur schwer sterilisierbar, was einen Einsatz der in WO-A-2005/098423 beschriebenen Messanordnung unter sterilen Messbedingungen erschwert. Auch ist der Einsatz der in der WO-A-2005/098423 beschriebenen Messanordnung in Form von in Laboren häufig bevorzugten Einwegartikeln aufgrund der vergleichsweise teuren Silber/Silberchlorid-Elektroden nachteilig. Im Übrigen wird bei dem in der WO-A-2005/098423 beschriebenen Messverfahren die Zellschicht auch nicht mittels Impedanzspektroskopie, sondern über ein Gleichspannungsmessverfahren analysiert.

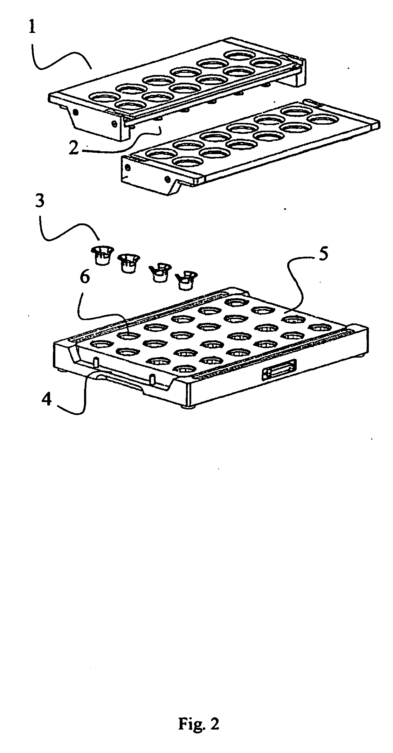

Eine Messanordnung, mittels derer eine Vielzahl von Membransystemen impedanzspektroskopisch auch unter sterilen Bedingungen analysiert werden können, ist das von der Anmelderin unter der Bezeichnung cellZscope^{®} vertriebene System, welches in der Figur 2 gezeigt ist. Dieses System besteht aus einer Bodenplatte aus Edelstahl, auf die ein mit Bohrungen versehener Kunststoffblock gelegt wird. Die Bohrungen in dem Kunststoffblock bilden zusammen mit der Oberfläche der Bodenplatte ein Behältnis, wobei mittels eines Gummiringes ein flüssigkeitsdichter Kontakt zwischen dem Kunststoffblock und der Bodenplatte sichergestellt wird. In die Behältnisse können dann Membransysteme, wie sie kommerziell beispielsweise von den Firmen Corning, Greiner Bio-One, BD Biosciences, Millipore oder Nunc erhältlich sind, eingehängt werden. Das cellZscope^{®}-System umfasst weiterhin eine Abdeckung mit einer Vielzahl von Elektroden, die, wenn der Kunststoffblock mit dieser Abdeckung abgedeckt wird, jeweils in die Membransysteme hineinragen. Der Nachteil dieses cellZscope^{®}-Systems besteht jedoch darin, dass die Reinigung des Systems nach Durchführung der jeweiligen Messungen aufgrund der Anwesenheit der Vielzahl von die Behältnisse bildenden Komponenten (Bodenplatte, Gummiringe, Kunststoffblock) unter Umständen vergleichsweise aufwendig ist. Auch können aufgrund der vorgegebenen Bohrungen in den Kunststoffplatten bei einer gegebenen Kunststoffplatte stets nur Membransysteme des gleichen Durchmessers analysiert werden.

Eine weitere Messanordnung wird in WO 2006/112870 offenbart.

Der vorliegenden Erfindung lag die Aufgabe zugrunde, die sich aus dem Stand der Technik ergebenden Nachteile zu überwinden.

Insbesondere lag der vorliegenden Erfindung die Aufgabe zugrunde, eine Vorrichtung zur Bestimmung der transzellularen Impedanz von auf einer permeablen Membran kultivierten Zellen bereitzustellen, die sich möglichst leicht reinigen lässt. Darüber hinaus sollte sich die Vorrichtung durch eine besonders hohe Flexibilität hinsichtlich der Membrandurchmesser der zu analysierenden Membransysteme auszeichnen.

Auch lag der vorliegenden Erfindung die Aufgabe zugrunde, eine Vorrichtung zur Bestimmung der transzellularen Impedanz von auf einer permeablen Membran kultivierten Zellen bereitzustellen, wobei die Komponenten der Vorrichtung auch über lange Zeiträume unter dem Einfluss von Zellkulturmedium haltbar und damit vielfach wiederverwendbar sind. Da Bestimmungen der transzellularen Impedanz in der Regel unter sterilen Bedingungen durchgeführt werden, soll sich die Vorrichtung insbesondere auch dadurch auszeichnen, dass die einzelnen Komponenten wiederholt sowohl in einem Ultraschallbad gereinigt als auch in einem Autoklaven sterilisiert werden können.

Einen Beitrag zur Lösung der eingangs genannten Aufgaben leistet eine Vorrichtung zur Bestimmung der transzellularen Impedanz von auf einer permeablen Membran kultivierten Zellen, umfassend eine oder mehrere Messzellen, wobei jede Messzelle
a) ein erstes Behältnis, in das ein zweites Behältnis umfassend die permeableMembran als Zellkultursubstrat eingesetzt werden kann, sowie
b) eine Abdeckung für das erste Behältnis umfassend eine Elektrode, wobei die Elektrode so an der Abdeckung angeordnet ist, dass, wenn das zweite Behältnis in dem ersten Behältnis eingesetzt ist, das zweite Behältnis zumindest teilweise mit einer leitenden Flüssigkeit gefüllt ist und das erste Behältnis mit der Abdeckung abgedeckt wird, die leitende Flüssigkeit in dem zweiten Behältnis in Kontakt mit der Elektrode treten kann,
umfasst, dadurch gekennzeichnet, dass mindestens 50 %, vorzugsweise mindestens 70 %, noch mehr bevorzugt mindestens 90 % und am meisten bevorzugt 100 % der dem Innenraum des ersten Behältnisses zugewandten Oberflächen des ersten Behältnisses aus einem elektrisch leitfähigen Material gebildet sind, wobei die Prozentangaben als Flächenprozente zu verstehen sind. Beträgt beispielsweise die gesamte, dem Innenraum des ersten Behältnisses zugewandte Oberfläche 16 cm², so sind mindestens 8 cm² (50 %) dieser Oberfläche aus einem elektrisch leitfähigen Material gebildet.

Dabei können die Behältnisse beispielsweise teilweise oder vollständig aus einem leitfähigen Material gebildet werden oder die inneren Oberflächen der Behältnisse können teilweise oder vollständig mit einem leitfähigen Material beschichtet werden. Besonders bevorzugt ist es, dass die Behältnisse vollständig aus einem leitfähigen Material, ganz besonders bevorzugt vollständig aus einem Metall oder einer metallischen Legierung, gebildet sind.

Gemäß einer ersten besonderen Ausführungsform der erfindungsgemäßen Vorrichtung ist das erste Behältnis zu mindestens 50 Gew.-%, besonders bevorzugt zu mindestens 70 Gew.-%, noch mehr bevorzugt zu mindestens 90 Gew.-%, noch mehr bevorzugt zu mindestens 95 Gew.-% und am meisten bevorzugt zu 100 Gew.-%, jeweils bezogen auf das Gesamtgewicht des ersten Behältnisses, aus einem Metall bzw. einer elektrisch leitfähigen, metallischen Legierung, beispielsweise aus Stahl, Gold oder Platin, besonders bevorzugt aus Stahl, am meisten bevorzugt aus Edelstahl, gebildet. Es werden demnach bei dieser besonderen Ausführungsform der erfindungsgemäßen Vorrichtung ganz besonders bevorzugt vollständig aus Edelstahl gebildete, topfförrnigeelektroden als erste Behältnisse eingesetzt.

Gemäß einer zweiten besonderen Ausführungsform der erfindungsgemäßen Vorrichtung sind mindestens 50 %, besonders bevorzugt mindestens 70 %, noch mehr bevorzugt mindestens 90 %, noch mehr bevorzugt mindestens 95 % und am meisten bevorzugt 100 % der dem Innenraum des ersten Behältnisses zugewandten Oberflächen des ersten Behältnisses mit einem Metall bzw. einer metallischen Legierung, beispielsweise mit Gold oder Platin, beschichtet.

Gemäß einer dritten besonderen Ausführungsform der erfindungsgemäßen Vorrichtung ist das erste Behältnis zu mindestens 50 Gew.-%, besonders bevorzugt zu mindestens 70 Gew.-%, noch mehr bevorzugt zu mindestens 90 Gew.-%, noch mehr bevorzugt zu mindestens 95 Gew.-% und am meisten bevorzugt zu 100 Gew.-%, jeweils bezogen auf das Gesamtgewicht des ersten Behältnisses, aus einem elektrisch leitfähigen Kunststoff gebildet.

Gemäß einer vierten besonderen Ausführungsform der erfindungsgemäßen Vorrichtung sind mindestens 50 %, besonders bevorzugt mindestens 70 %, noch mehr bevorzugt mindestens 90 %, noch mehr bevorzugt mindestens 95 % und am meisten bevorzugt 100 % der dem Innenraum des ersten Behältnisses zugewandten Oberflächen des ersten Behältnisses mit einem elektrisch leitfähigen Kunststoff beschichtet.

Gemäß einer besonderen Ausführungsform der erfindungsgemäßen Behältnisse ist der Boden der ersten Behältnisse im Wesentlichen planar. Insbesondere weist der Boden der ersten Behältnisse keine in der US 2007/0292837 A1 als "Picowell" bezeichneten Vertiefungen aus, in denen Zellen kultiviert und individualisiert analysiert werden können.

Überraschenderweise, dafür aber nicht minder vorteilhaft, wurde festgestellt, dass sich die Impedanz von Zellschichten in Messsystemen wie etwa dem cellZscope^{®}-System auch ermitteln lassen, wenn als untere Behältnisse nicht Behältnisse eingesetzt werden, in denen ausschließlich der Boden oder ein Teil des Bodens aus einem elektrisch leitfähigen Material (Stahl) besteht, sondern Behältnisse, die vorzugsweise vollständig aus einem elektrisch leitfähigen Material gefertigt sind oder deren innere Oberflächen vorzugsweise vollständig mit einem elektrisch leitfähigen Material beschichtet sind. Im Hinblick auf die Tatsache, dass bei derartigen Behältnissen die Verteilung des elektrischen Feldes gänzlich verschieden ist von einem Behältnis, bei dem ausschließlich der Boden oder ein Teil des Bodens aus einem elektrisch leitfähigen Material besteht, konnte der Fachmann insbesondere nicht erwarten, dass sich dennoch die transzellulare Impedanz präzise ermitteln lässt. Es ist somit nicht erforderlich, die Behältnisse aus mehreren, über Gummidichtungen flüssigkeitsdicht zu verbindenden Komponenten zu fertigen. Vielmehr können einstückige Behältnisse eingesetzt werden, die sich vergleichsweise leicht reinigen und sterilisieren lassen.

Als elektrisch leitfähige Kunststöffe, aus denen die inneren Behältnisse gemäß der dritten besonderen Ausführungsform der erfindungsgemäßen Vorrichtung zumindest teilweise gebildet werden können oder mit denen gemäß der vierten besonderen Ausführungsform die dem Innenraum des ersten Behältnisses zugewandten Oberflächen des ersten Behältnisses zumindest teilweise beschichtet werden können, kommen beispielsweise *cis*-Polyacetylen, trans-Polyacetylen, Poly*(para-*phenylen), Polythiophen, Polypyrrol, Poly(para-phenylen-vinylen) oder Polyanilin in Betracht, wobei insbesondere zur Beschichtung der dem Innenraum des ersten Behältnisses zugewandten Oberflächen des ersten Behältnisses der Einsatz von Polythiophenen, beispielsweise von Poly(3,4-ethylendioxythiphen) besonders bevorzugt ist. In diesem Zusammenhang ist es insbesondere bevorzugt, die Poly(3,4-ethylendioxythiophen), welches nach seiner Herstellung aus 3,4-Ethylendioxythiophen durch oxidative Polymerisation als Polykation vorliegt, in Kombination mit Polyanionen, beispielsweise in Kombination mit Polysulfonaten, wie Poly(styrolsulfonat), als Beschichtungsmaterial einzusetzen. Derartige leitfähige Polymerzusammensetzungen auf der Basis von Poly(3,4-ethylendioxythiphen) bzw. Poly(3,4-ethylendioxythiophen)/Poly(styrolsulfonat)-Mischungen sind beispielsweise in Form von Polymerdispersionen von der H. C. Starck Clevios GmbH, Gosslar, Deutschland, erhältlich.

Da die in der erfindungsgemäßen Vorrichtung einzusetzenden Membransysteme (= zweite Behältnisse umfassend die permeable Membran als Zellkultursubstrat) üblicherweise rund sind, weisen die ersten Behältnisse vorzugsweise ebenfalls einen kreisrunden Querschnitt auf. Hinsichtlich der genauen Ausgestaltung der Messzellen und deren Anordnung in der erfindungsgemäßen Vorrichtung sind nun unterschiedliche Ausführungsformen möglich.

Gemäß einer ersten besonderen Ausgestaltung der erfindungsgemäßen Vorrichtung umfasst diese mindestens 6 Messzellen, vorzugsweise mindestens 12, mindestens 16, mindestens 20, mindestens 24, mindestens 48 oder mindestens 96 Messzellen, beispielsweise genau 6, 12, 16, 20, 24, 48 oder 96 Messzellen, ganz besonders bevorzugt genau 24 Messzellen,
- wobei die mindestens 6 ersten Behältnisse auf einer gemeinsamen Bodenplatte angeordnet sind,
- wobei eine gemeinsame Abdeckung für die mindestens 6 ersten Behältnisse vorgesehen ist,
- wobei die gemeinsame Abdeckung mindestens 6 Elektroden umfasst, und
- wobei die mindestens 6 Elektroden so an der gemeinsamen Abdeckung angeordnet sind, dass, wenn jeweils das zweite Behältnis in dem jeweils ersten Behältnis eingesetzt ist, das jeweils zweite Behältnis zumindest teilweise mit einer leitenden Flüssigkeit gefüllt ist und die mindestens 6 ersten Behältnisse mit der gemeinsamen Abdeckung abgedeckt werden, die leitende Flüssigkeit in jedem der zweiten Behältnisse in Kontakt mit einer Elektrode treten kann.

Diese erste besondere Ausgestaltung der erfindungsgemäßen Vorrichtung leitet sich vom cellZscope^{®}-System ab, wobei im Unterschied zu diesem die ersten Behältnisse nicht durch das Zusammenwirken der Bodenplatte mit den Bohrungen im Kunststoffblock gebildet werden, sondern aus den vorzugsweise einstückigen ersten Behältnissen, welche vorzugsweise ausschließlich aus einem elektrisch leitfähigen Material, insbesondere aus Metall bzw. einer metallischen Legierung, gebildet oder deren dem Innenraum zugewandte Oberflächen vorzugsweise vollständig mit einem elektrisch leitfähigen Material beschichtet sind.

Gemäß einer Variante der erfindungsgemäßen Vorrichtung, bei der die Elektroden in bzw. an der Abdeckung einzeln kontaktiert werden, ist die gemeinsame Bodenplatte vorzugsweise aus einem elektrisch leitfähigen Material, wie etwa Stahl, vorzugsweise Stahl, ganz besonders bevorzugt Edelstahl, gebildet oder zumindest mit einem Metall bzw. einer metallischen Legierung, wie beispielsweise Gold oder Platin, oder einem elektrisch leitfähigen Kunststoff, wie etwa einem Polythiophen, beschichtet, wobei der Einsatz einer Stahlplatte, insbesondere einer Edelstahlplatte, als Bodenplatte besonders bevorzugt ist.

Gemäß einer anderen Variante der erfindungsgemäßen Vorrichtung, bei der die dem Innenraum des ersten Behältnisses zugewandten Oberflächen der ersten Behältnisse einzeln kontaktiert werden, ist die gemeinsame Bodenplatte vorzugsweise aus einem elektrisch nicht leitfähigen Material, beispielsweise aus herkömmlichem Kunststoff, gefertigt.

Die Größe dieser Bodenplatte hängt davon ab, wie groß die Anzahl an Messzellen in der erfindungsgemäßen Vorrichtung ist. Üblicherweise jedoch hat die Bodenplatte eine Länge in einem Bereich von 15 bis 40 cm, besonders bevorzugt in einem Bereich von 20 bis 35 cm und am meisten bevorzugt in einem Bereich von 25 bis 30 cm, eine Breite in einem Bereich von 10 bis 30 cm, besonders bevorzugt in einem Bereich von 12,5 bis 25 cm und am meisten bevorzugt in einem Bereich von 15 bis 20 cm und eine Dicke in einem Bereich von 1 bis 15 mm, besonders bevorzugt in einem Bereich von 2 bis 10 mm und am meisten bevorzugt in einem Bereich von 3 bis 5 mm.

Die mindestens 6 ersten Behältnisse sind auf dieser gemeinsamen Bodenplatte angeordnet, wobei die Behältnisse vorzugsweise so auf der Bodenplatte befestigt sind, dass die Behältnisse bei Bewegungen der Bodenplatte, etwa wenn die Messvorrichtung in einen Brutschrank platziert wird, nicht auf der Bodenplatte verrutschen. In diesem Zusammenhang kann es vorteilhaft sein, die ersten Behältnisse auf der Unterseite des Bodens mit Schraubgewinden und die Bodenplatte mit entsprechenden Gewindebohrungen zu versehen, so dass die Behältnisse auf die gemeinsame Bodenplatte aufgeschraubt werden können. Denkbar ist auch, die ersten Behältnisse beispielsweise mittels Magneten, welche an der Bodenplatte und/oder den ersten Behältnissen angebracht sind, auf der Bodenplatte zu befestigen oder aber die Bodenplatte mit einfachen Bohrungen zu versehen, in welche die ersten Behältnisse, gegebenenfalls über einen am Boden der ersten Behältnisse befestigten Dom, bis zu einer bestimmten Tiefe eingesetzt werden können. Weiterhin können beispielsweise die ersten Behältnisse mittels Federkontakte auf die Bodenplatte aufgesetzt werden. Aufgrund dieser Befestigungsmethoden können die ersten Behältnisse besonders einfach auf einer Basisplatte befestigt bzw. von dieser entfernt werden, was die Handhabung zum regelmäßigen Reinigen erheblich vereinfacht.

Die Anordnung der Gewindebohrungen, der Magneten oder der einfachen Bohrungen auf der Bodenplatte erfolgt vorzugsweise in äquidistanten Abständen, besonders bevorzugt in Form einer 48 ×2-, einer 24 × 4-, einer 16 × 6- oder einer 12 ×8-Koordination (im Falle einer Vorrichtung mit 96 Messzellen), einer 24 ×2- , einer 16 × 3-, 12 × 4- oder einer 8 x 6-Koordination (im Falle einer Vorrichtung mit 48 Messzellen), einer 12 × 2-, einer 8 × 3- oder einer 6 × 4-Koordination (im Falle einer Vorrichtung mit 24 Messzellen), einer 10 × 2- oder einer 5×4-Koordination (im Falle einer Vorrichtung mit 20 Messzellen), einer 8 × 2- oder einer 4 × 4-Koordination (im Falle einer Vorrichtung mit 16 Messzellen), einer 4 × 3- oder einer 6 × 2-Koordination (im Falle einer Vorrichtung mit 12 Messzellen) oder einer 3 × 2-Koordination (im Falle einer Vorrichtung mit 6 Messzellen).

Im Zusammenhang mit dieser ersten besonderen Ausgestaltung der erfindungsgemäßen Vorrichtung handelt es sich bei den ersten Behältnissen um Behältnisse aus Stahl, besonders bevorzugt um Behältnisse auch Edelstahl. Diese Behältnisse weisen vorzugsweise eine Wandstärke in einem Bereich von 0,1 bis 10,0 mm, besonders bevorzugt in einem Bereich von 1 bis 6 mm und am meisten bevorzugt in einem Bereich von 2 bis 4 mm und eine Höhe in einem Bereich von 10 bis 40 mm, besonders bevorzugt in einem Bereich von 15 bis 30 mm und am meisten bevorzugt in einem Bereich von 20 bis 25 mm auf.

Der Durchmesser des Innenraums der Behältnisse hängt von dem Membrandurchmesser des zweiten Behältnisses ab.

Gemäß einer ersten Variante dieser ersten besonderen Ausgestaltung der erfindungsgemäßen Vorrichtung weisen die ersten Behältnisse einen solchen Durchmesser des Innenraumes auf, dass in diese ersten Behältnisse zweite Behältnisse mit einem Membrandurchmesser in einem Bereich von 20 bis 26 mm, besonders bevorzugt von etwa 24 mm eingesetzt werden können. In diesem Zusammenhang ist es bevorzugt, dass der Durchmesser des Innenraumes der ersten Behältnisse in einem Bereich von 26 bis 52 mm, besonders bevorzugt in einem Bereich von 32 bis 39 mm liegt. Für derartige erste Behältnisse geeignete Membransysteme sind beispielsweise
- von der Firma Corning Inc., Acton, USA unter der Bezeichnung TRANSWELL^{®}INSERTS unter den Produktnummem 3412, 3414, 3428, 3450, 3452, 3491 und 3492,
- von der Firma Greiner Bio-One, Frickenhausen, Deutschland, unter der Bezeichnung ThinCert^{®} unter den Produktnummem 657610, 657630, 657631, 657638, 657640 und 657641,
- von der Firma BD Biosciences, San Jose, USA, unter der Bezeichnung BD FALCON^{®} unter den Produktnummem 353493, 353090, 353102, 353092, 353091, 353093, 354442, 354580, 354540, 354544, 354570, 354567, 354576, 354472, 354440, 354443 und 354481,
- von der Firma Millipore, Billerica, USA, unter der Bezeichnung MILLICEL HANGING INSERTS unter den Produktnummem PIHT30R48, PIRP30R48, PISP30R48, PIMP30R48 und PIEP30R48, oder
- von der Firma Thermo Fisher Scientific, Rosklide, Dänemark, unter der Bezeichnung NUNC CC INSERTS unter den Produktnummem 140640, 140642, 140644, 140660, 140663, 140668, 161395, 137044, 137060, 137435 oder 137508

### erhältlich.

Gemäß einer zweiten Variante dieser ersten besonderen Ausgestaltung der erfindungsgemäßen Vorrichtung weisen die ersten Behältnisse einen solchen Durchmesser des Innenraums auf, dass in diese ersten Behältnisse zweite Behältnisse mit einem Membrandurchmesser in einem Bereich von 10 bis 14 mm, besonders bevorzugt von etwa 12 mm eingesetzt werden können. In diesem Zusammenhang ist es bevorzugt, dass der Durchmesser des Innenraums der ersten Behältnisse in einem Bereich von 14 bis 28 mm, besonders bevorzugt in einem Bereich von 20 bis 25 mm liegt. Für derartige erste Behältnisse geeignete Membransysteme sind beispielsweise
- von der Firma Corning Inc., Acton, USA unter der Bezeichnung TRANSWELL^{®}INSERTS unter den Produktnummem 3460, 3462, 3401, 3402, 3403, 3493 und 3494,
- von der Firma Greiner Bio-One, Frickenhausen, Deutschland, unter der Bezeichnung ThinCert^{®} unter den Produktnummem 665640, 665641, 665610, 665630, 665631 und 665638,
- von der Firma BD Biosciences, San Jose, USA, unter der Bezeichnung BD FALCON^{®} unter den Produktnummem 353494, 353180, 353103, 353292, 353181, 353182, 354490, 354581, 354565, 354571, 354568, 354574, 354473 und 354492,
- von der Firma Millipore, Billerica, USA, unter der Bezeichnung MILLICEL HANGING INSERTS unter den Produktnummem PIHT15R48, PIRP15R48, PISP15R48, PIMP15R48 und PIEP15R48, oder
- von der Firma Thermo Fisher Scientific, Rosklide, Dänemark, unter der Bezeichnung NUNC CC INSERTS unter den Produktnummem 140652, 140654 und 140656

### erhältlich.

Gemäß einer dritten Variante dieser ersten besonderen Ausgestaltung der erfindungsgemäßen Vorrichtung weisen die ersten Behältnisse einen solchen Durchmesser des Innenraums auf, dass in diese ersten Behältnisse zweite Behältnisse mit einem Membrandurchmesser in einem Bereich von 5,5 bis 8,0 mm, besonders bevorzugt von etwa 6,5 mm eingesetzt werden können. In diesem Zusammenhang ist es bevorzugt, dass der Durchmesser des Innenraums der ersten Behältnisse in einem Bereich von 8 bis 25 mm, besonders bevorzugt in einem Bereich von 14 bis 20 mm liegt. Für derartige erste Behältnisse geeignete Membransysteme sind beispielsweise
- von der Firma Corning Inc., Acton, USA unter der Bezeichnung TRANSWELL^{®}INSERTS unter den Produktnummem 3470, 3472, 3413, 3415, 3421, 3422, , 3495 und 3496,
- von der Firma Greiner Bio-One, Frickenhausen, Deutschland, unter der Bezeichnung ThinCert^{®} unter den Produktnummem 662640, 662641, 662610, 662630, 662631 und 662638,
- von der Firma BD Biosciences, San Jose, USA, unter der Bezeichnung BD FALCON^{®} unter den Produktnummem 353495, 353095, 353104, 353492, 353096, 353097, 354436, 354437, 354444, 354482, 354541, 354591, 354545, 354572, 354569, 354575, 354578, 354474 und 354445,
- von der Firma Millipore, Billerica, USA, unter der Bezeichnung MILLICEL HANGING INSERTS unter den Produktnummem PIHT12R48, PIRP12R48, PISP12R48, PIMP12R48 und PIEP12R48, oder
- von der Firma Thermo Fisher Scientific, Rosklide, Dänemark, unter der Bezeichnung NUNC CC INSERTS unter den Produktnummem 140620, 140627, 140629, 162243, 136935, 137052, 137370 und 137443

### erhältlich.

Dabei ist es möglich, auch erste Behältnisse mit unterschiedlichem innerem Durchmesser auf der gemeinsamen Bodenplatte anzuordnen. Im Gegensatz zum cellZscope^{®}-System ist es somit mit dieser ersten besonderen Ausgestaltung der erfindungsgemäßen Vorrichtung auch möglich, zugleich die Impedanz von Zellschichten zu bestimmen, die auf Membransystemen mit unterschiedlichen Membrandurchmessern kultiviert wurden.

Neben der gemeinsamen Bodenplatte und den vorstehend beschriebenen ersten Behältnissen umfasst die erfindungsgemäße Vorrichtung dieser ersten besonderen Ausgestaltung weiterhin eine gemeinsame Abdeckung für die mindestens 6 ersten Behältnisse, welche mindestens 6 Elektroden umfasst. Vorzugsweise dient dabei als gemeinsame Abdeckung eine Kunststoffplatte, vorzugsweise eine transparente Kunststoffplatte, an der die Elektroden senkrecht derart angebracht sind, dass sie, wenn die ersten Behältnisse mit der gemeinsamen Abdeckung abgedeckt werden, mit der elektrisch leitfähigen Flüssigkeit in den jeweiligen Membransystemen (= zweite Behältnisse) in Kontakt treten. Eine in diesem Zusammenhang bevorzugte gemeinsame Abdeckung ist die bereits im cellZscope^{®}-System eingesetzte Abdeckung. Diese gemeinsame Abdeckung kann weiterhin mit Öffnungen in der Nähe der Elektroden versehen sein, über die Flüssigkeiten in das erste und/oder zweite Behältnis eingefiillt oder aber aus dem ersten und/oder zweiten Behältnis entnommen werden können, ohne das hierzu die gemeinsame Abdeckung entfernt werden muss. Auch solche Öffnungen sind bereits im cellZscope^{®}-System vorgesehen.

Neben der gemeinsamen Bodenplatte, den ersten Behältnissen und der gemeinsamen, die Elektroden umfassenden Abdeckung kann die erfindungsgemäße Vorrichtung weiterhin Leiterbahnen, beispielsweise aus Edelstahl, umfassen, welche die Elektroden an bzw. in der gemeinsamen Abdeckung oder aber die dem Innenraum der ersten Behältnisse zugewandten Oberflächen kontaktieren, sowie eine entsprechende Schnittstelle, über die beispielsweise eine sinusförmige Wechselspannung kleiner Amplitude an die jeweiligen Elektroden angelegt und der Widerstand über die Zellschicht des entsprechenden Membransystems gemessen werden kann.

Gemäß einer zweiten besonderen Ausgestaltung der erfindungsgemäßen Vorrichtung umfasst diese mindestens 6 Messzellen,
- wobei die mindestens 6 ersten Behältnisse in Form einer Multiwellplatte vorliegen,
- wobei eine gemeinsame Abdeckung für die mindestens 6 ersten Behältnisse vorgesehen ist,
- wobei die gemeinsame Abdeckung mindestens 6 Elektroden umfasst, und
- wobei die mindestens 6 Elektroden so an der gemeinsamen Abdeckung angeordnet sind, dass, wenn jeweils das zweite Behältnis in dem jeweils ersten Behältnis eingesetzt ist, das jeweils zweite Behältnis zumindest teilweise mit einer leitenden Flüssigkeit gefüllt ist und die mindestens 6 ersten Behältnisse mit der gemeinsamen Abdeckung abgedeckt werden, die leitende Flüssigkeit in jedem der zweiten Behältnisse in Kontakt mit einer Elektrode treten kann.

Im Gegensatz zur ersten besonderen Ausgestaltung der erfindungsgemäßen Vorrichtung umfasst diese zweite besondere Ausgestaltung keine gemeinsame Bodenplatte, auf der voneinander getrennte erste Behältnisse angebracht werden können, sondern eine Multiwellplatte. Dabei ist es besonders bevorzugt, dass die Multiwellplatte entweder zu mindestens 50 Gew.-%, noch mehr bevorzugt zu mindestens 70 Gew.-%, noch mehr bevorzugt zu mindestens 90 Gew.-%, noch mehr bevorzugt zu mindestens 95 Gew.-% und am meisten bevorzugt zu 100 Gew.-%, bezogen auf das Gesamtgewicht der Multiwellplatte, aus einem elektrisch leitfähigen Material, vorzugsweise aus einem elektrisch leitfähigen Kunststoff, besteht oder dass mindestens 50 %, noch mehr bevorzugt mindestens 70 %, noch mehr bevorzugt mindestens 90 %, noch mehr bevorzugt mindestens 95 % und am meisten bevorzugt 100 % der Oberfläche in den jeweiligen Vertiefungen der Multiwellplatte mit einem elektrisch leitfähigen Material, vorzugsweise mit einem elektrisch leitfähigen Kunststoff, beschichtet sind. Sind lediglich die Oberflächen der Vertiefungen mit einem elektrisch leitfähigen Material beschichtet, so können die Multiwellplatten auf herkömmlichen Multiwellplatten, insbesondere auf herkömmlichen 6-Well-, 12-Well-, 24-Well-Platten basieren, wie sie etwa
- von der Firma Corning Inc., Acton, USA unter den Produktnummem 3506, 3516, 3512, 3513, 3524, 3526 und 3527,
- von der Firma Greiner Bio-One, Frickenhausen, Deutschland, unter den Produktnummem 657160, 657185, 647960, 665102, 665180, 665110, 665980, 704160, 662102 und 662960,
- von der Firma BD Biosciences, San Jose, USA, unter der Bezeichnung BD FALCON^{®} unter den Produktnummem 351146, 353046, 353934, 352224, 351143, 353043, 353225, 353935, 351147, 353047 und 353226, oder
- von der Firma Millipore, Billerica, USA, unter den Produktnummem PIMWS0650, PIMWS1250 und PIMWS2450,

erhältlich sind. Die Oberflächen derartiger Multiwellplatten können beispielsweise mit leitfähigen Polythiophenen beschichtet werden, wobei hierzu insbesondere die Eingangs beschriebenen Polymerzusammensetzungen auf der Basis von Poly(3,4-ethylendioxythiphen) bzw. Poly(3,4-ethylendioxythiophen)/Poly(styrolsulfonat)-Mischungen eingesetzt werden können.

Gemäß einer ersten Variante dieser zweiten besonderen Ausgestaltung der erfindungsgemäßen Vorrichtung ist die Multiwellplatte eine 6-Wellplatte, in deren erste Behältnisse zweite Behältnisse mit einem Membrandurchmesser in einem Bereich von 20 bis 26 mm, besonders bevorzugt von etwa 24 mm, eingesetzt werden können, wobei als Membransysteme wiederum diejenigen bevorzugt sind, die bereits vorstehend im Zusammenhang mit der ersten Variante der ersten besonderen Ausgestaltung der erfindungsgemäßen Vorrichtung als bevorzugte Membransysteme genannt wurden.

Gemäß einer zweiten Variante dieser zweiten besonderen Ausgestaltung der erfindungsgemäßen Vorrichtung ist die Multiwellplatte eine 12-Wellplatte, in deren erste Behältnisse zweite Behältnisse mit einem Membrandurchmesser in einem Bereich von 10 bis 14 mm, besonders bevorzugt von etwa 12 mm, eingesetzt werden können, wobei als Membransysteme wiederum diejenigen bevorzugt sind, die bereits vorstehend im Zusammenhang mit der zweiten Variante der ersten besonderen Ausgestaltung der erfindungsgemäßen Vorrichtung als bevorzugte Membransysteme genannt wurden.

Gemäß einer dritten Variante dieser zweiten besonderen Ausgestaltung der erfindungsgemäßen Vorrichtung ist die Multiwellplatte eine 12-Wellplatte, in deren erste Behältnisse zweite Behältnisse mit einem Membrandurchmesser in einem Bereich von 5,5 bis 8,0 mm, besonders bevorzugt von etwa 6,5 mm, eingesetzt werden können, wobei als Membransysteme wiederum diejenigen bevorzugt sind, die bereits vorstehend im Zusammenhang mit der dritten Variante der ersten besonderen Ausgestaltung der erfindungsgemäßen Vorrichtung als bevorzugte Membransysteme genannt wurden.

Neben der Multiwellplatte umfasst die erfindungsgemäße Vorrichtung auch dieser zweiten besonderen Ausgestaltung weiterhin eine gemeinsame Abdeckung für die mindestens 6 ersten Behältnisse, welche mindestens 6 Elektroden.

Gemäß einer dritten besonderen Ausgestaltung der erfindungsgemäßen Vorrichtung umfasst diese einen Metallblock, vorzugsweise einem Block aus Edelstahl, in den Bohrungen eingebracht werden, die als erste Behältnisse dienen. Hinsichtlich der Länge und Bereite des Metallblocks wird auf die Angaben im Zusammenhang mit der Länge und Breite der in der ersten besonderen Ausgestaltung der erfindungsgemäßen Vorrichtung vorgesehenen Bodenplatte verwiesen, wobei die Dicke des Metallblocks vorzugsweise in einem Bereich von 1 bis 10 cm, besonders in einem Bereich von 1,5 bis 7 cm und am meisten bevorzugt in einem Bereich von 2 bis 5 cm liegt. Der Durchmesser und die Tiefe der Bohrungen hängt vom Membrandurchmesser der in die Bohrungen einzusetzenden Membransysteme ab, wobei hinsichtlich des Durchmessers der Bohrungen wiederum auf die Angaben zum Durchmesser des Innenraumes der ersten Behältnisse im Zusammenhang mit der ersten, zweiten und dritten Variante der ersten besonderen Ausgestaltung der erfindungsgemäßen Vorrichtung verwiesen wird.

Einen Beitrag zur Lösung der eingangs genannten Aufgaben leistet auch ein Verfahren zur Bestimmung der transzellularen Impedanz von auf einer permeablen Membran kultivierten Zellen, umfassend die Verfahrensschritte
i) Bereitstellung einer erfindungsgemäßen Vorrichtung, wie sie vorstehend beschrieben wurde;
ii) Einsetzten mindestens eines zweiten Behältnisses umfassend eine permeable Membran als Zellkultursubstrat, auf der Zellen kultiviert sind, in mindestens eines der ersten Behältnisse;
iii) Befüllen des zweiten und des ersten Behältnisses mit einer elektrisch leitfähigen Flüssigkeit, wobei die Flüssigkeit im ersten Behältnis mindestens bis zu einer Füllhöhe, die bis zur permeablen Membran reicht, mit der Flüssigkeit befüllt wird;
iv) Abdecken des mindestens einen ersten Behältnisses mit der die Elektroden umfassenden Abdeckung;
v) Bestimmung der Impedanz der Zellschicht.

Im Verfahrensschritt i) des erfindungsgemäßen Verfahrens wird zunächst eine erfindungsgemäße Vorrichtung, wie sie vorstehend beschrieben wurde, bereitgestellt. Sodann wird im Verfahrensschritt ii) mindestens ein zweites Behältnis umfassend eine permeable Membran als Zellkultursubstrat, auf der Zellen kultiviert sind, in mindestens eines der ersten Behältnisse eingesetzt, wobei als zweites Behältnis diejenigen Membransysteme bevorzugt sind, die bereits eingangs im Zusammenhang mit der erfindungsgemäßen Vorrichtung als bevorzugte Membransysteme beschrieben wurden.

Im Verfahrensschritt iii) des erfindungsgemäßen Verfahrens wird dann das zweite und das ersten Behältnis mit einer elektrisch leitfähigen Flüssigkeit, beispielsweise einem Nährmedium oder einer Salzlösung, befüllt, wobei die Flüssigkeit im ersten Behältnis mindestens bis zu einer Füllhöhe, die bis zur permeablen Membran reicht, mit der Flüssigkeit befüllt wird. Gegebenenfalls kann das erste Behältnis zumindest teilweise auch vor dem Einsetzten des zweiten Behältnisses mit der Flüssigkeit befüllt werden. Weiterhin kann auch das zweite Behältnis vor dem Einsetzen in das erste Behältnis zumindest teilweise bereits mit der Flüssigkeit befüllt werden.

Nachdem im Verfahrensschritt iv) das mindestens eine erste Behältnis mit der die Elektroden umfassenden Abdeckung abgedeckt wurde, wird im Verfahrensschritt v) des erfindungsgemäßen Verfahrens die Impedanz der Zellschicht bestimmt. Dazu wird eine beispielsweise sinusförmige Wechselspannung kleiner Amplitude angelegt, deren Frequenz im Verlaufe der Messung verändert wird. Frequenzabhängig wird die Impedanz des Systems bestimmt. Das Prinzip der Impedanzspektroskopie beruht dabei auf der Bestimmung der elektrischen Impedanz der auf dem zu untersuchenden Membransystems kultivierten Zellen als Funktion der Frequenz des Erregersignals. Da es sich bei diesem Signal um eine Wechselspannung handelt, wird der elektrische Widerstand auch als Scheinwiderstand oder Impedanz bezeichnet. Zum Anlegen der sinusförmigen Wechselspannung und zur Bestimmung der elektrischen Parameter der Zellschicht können beispielsweise sogenannte *Impedance Gain Phase Analyzer* mit integriertem Frequenzgenerator verwendet werden.

Aus dem Impedanzspektrum können über entsprechende, rechnergestützte Analysesysteme der transzellulare Widerstand sowie die Kapazität einer Zellschicht ermittelt werden, wobei der transzellulare Widerstand Aussagen über die Permeabilität der Zellschicht für Ionen ermöglicht, während aus der Kapazität der Zellschicht Rückschlüsse über die Zellmorphologie möglich sind. Einzelheiten hierzu können beispielsweise Wegener et al., "Barrier function of porcine choroid plexus epithelial cells is modulated by cAMP-dependent pathways in vitro", Brain Research 853 (2000), Seiten 115 bis 124, entnommen werden.

Einen Beitrag zur Lösung der eingangs genannten Aufgaben leistet auch die Verwendung der erfindungsgemäßen Vorrichtung zur Bestimmung der transzellularen Impedanz von auf einer permeablen Membran kultivierten Zellen.

Die Erfindung wird nun anhand nicht limitierender Zeichnungen weiter erläutert.
Fig. 1 zeigt das Grundprinzip der Bestimmung der Impedanz Z(ω) von auf einem Membransystem kultivierten Zellen.
Fig. 2 zeigt das bereits bekannte cellZscope^{®}-System. Die Messvorrichtung umfasst einen mit Bohrungen versehenen Kunststoffblock 5, der auf einer Metallplatte 4 ruht. Die Wandung der Bohrung sowie die Metallplatte bilden dabei die ersten Behältnisse 6, in welche die entsprechenden Membransysteme 3 als zweite Behältnisse eingesetzt werden können. Mittels einer gemeinsamen Abdeckung 1, welche die Gegenelektroden 2 aufweist, können die ersten Behältnisse 6 derart abgedeckt werden, dass die Gegenelektroden 2 in die Flüssigkeit, welche sich in den Membransystemen 3 befindet, eingetaucht werden.

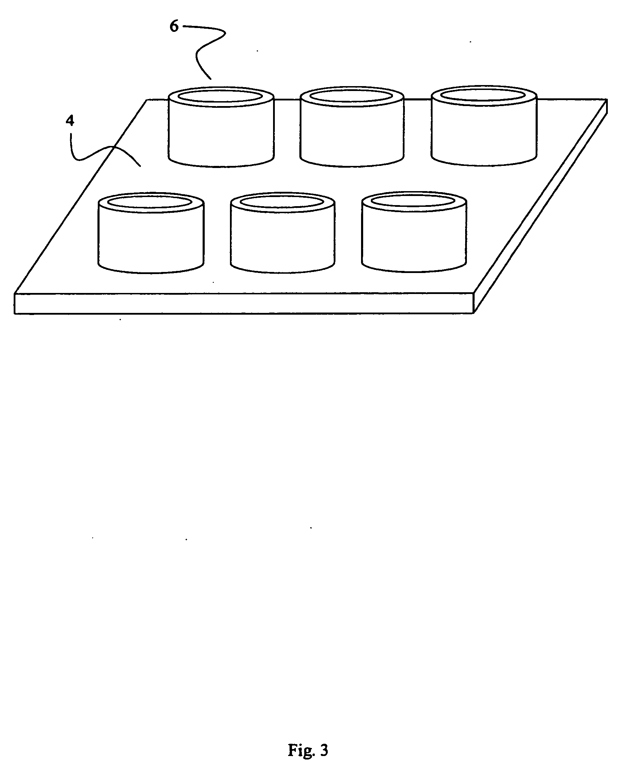

Fig. 3 zeigt die erste besondere Ausgestaltung der erfindungsgemäßen Vorrichtung, welche eine Bodenplatte 4, welche vorzugsweise aus Edelstahl gefertigt ist, umfasst. Auf der Bodenplatte 4 sind erste Behältnisse 6 befestigt, welche ebenfalls vorzugsweise aus Edelstahl gefertigt sind und in welche die in der Figur 2 gezeigten Membransysteme 3 eingesetzt werden können.

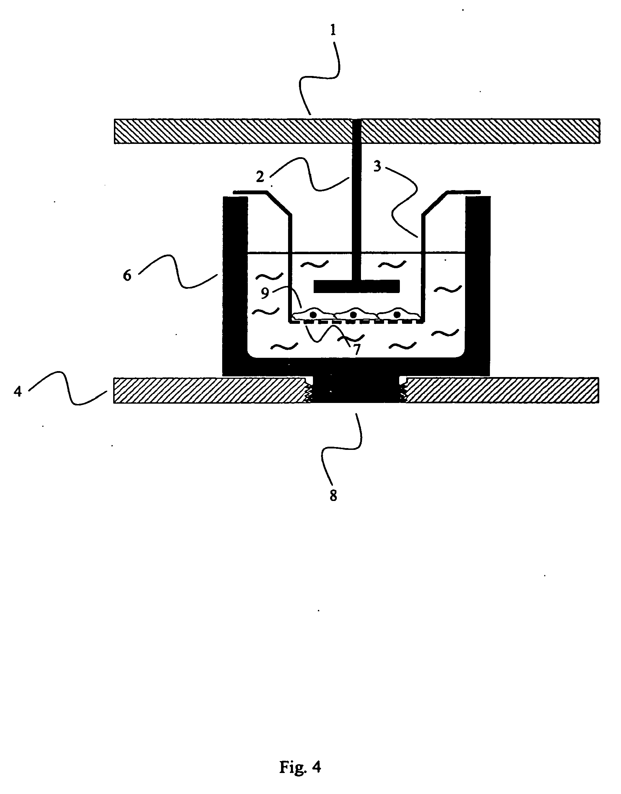

Fig. 4 zeigt, wie die ersten Behältnisse 6 über ein am Boden des Behältnisses 6 angebrachtes Gewinde 8 in entsprechende Gewindebohrungen in der Bodenplatte 4 eingeschraubt werden können. Zu sehen ist in der Figur 4 auch, wie ein zweites Behältnis 3 in Form eines eine permeable Membran 7 als Kultursubstrat umfassenden Membransystems, im dem Zellen 9 kultiviert wurden, in dem ersten Behältnis 6 eingesetzt und sowohl das erste Behältnis 6 als auch das zweite Behältnis 3 mit einer elektrisch leitfähigen Flüssigkeit befüllt sind. Bei der Messung der Impedanz der auf der permeablen Membran 7 kultivierten Zellen 9 ragt die Elektrode 2 in die elektrisch leitfähige Flüssigkeit, welche sich im zweiten Behältnis 3 befindet, hinein.

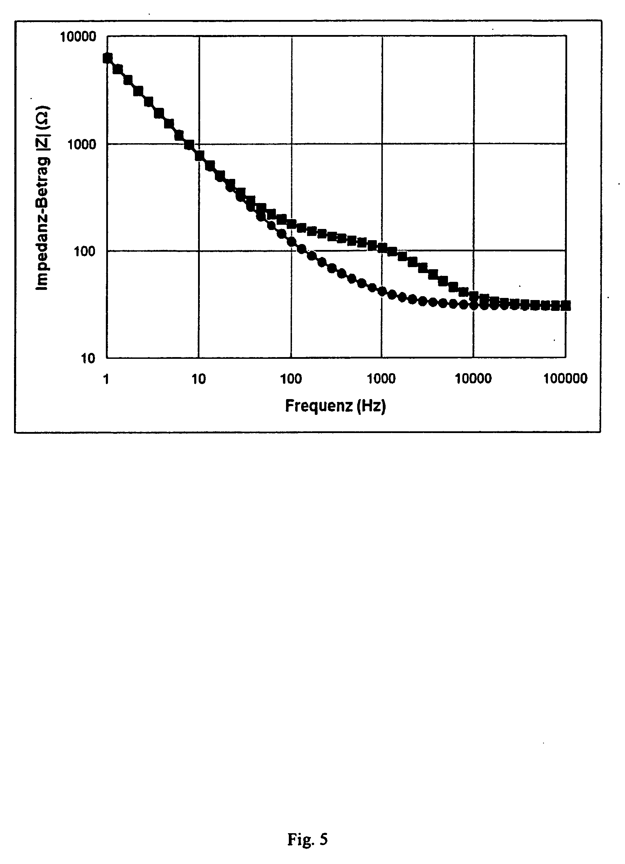

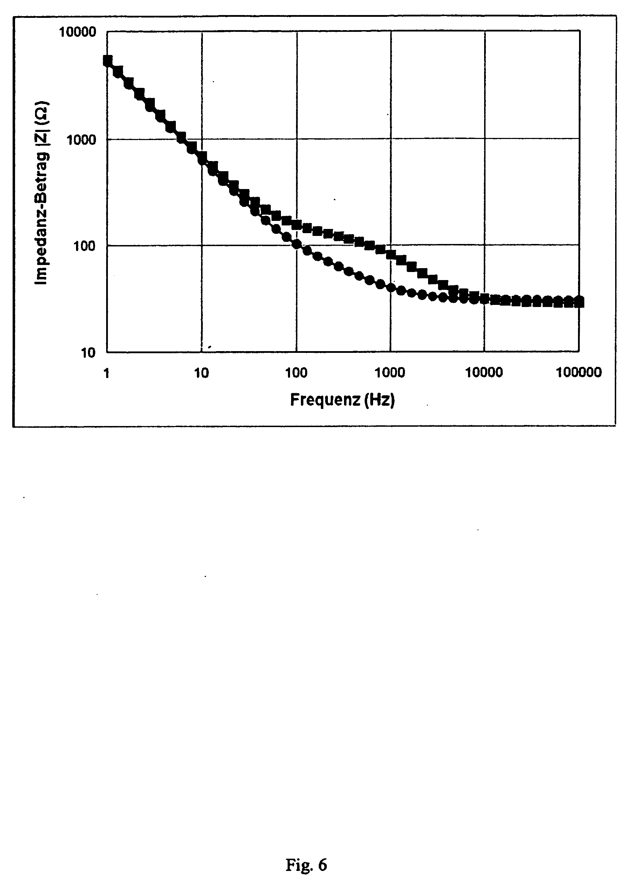

Die Figuren 5 und 6 zeigen ein mittels einer herkömmlichen (Figur 5) und einer erfindungsgemäßen (Figur 6) Vorrichtung aufgenommenes Impedanzspektrum einer barrierebildenden Zellschicht.

### BEISPIEL

MDCKII-Zellen (ECACC) werden auf Corning 3401 Inserts als Membransystem kultiviert. Mittels einer herkömmlichen Messvorrichtung (herkömmliches cellZscope^{®}-System der nanoAnalytics GmbH) sowie mittels einer erfindungsmä-βen Vorrichtung gemäß den Figuren 3 und 4 umfassend Edelstahltöpfchen als erste Behältnisse 6 wird jeweils ein Impedanzspektrum aufgenommen (siehe die Figuren 5 (cellZscope^{®}-System) und 6 (erfindungsgemäße Vorrichtung). Die mit Kreisen markierten Datensätze zeigen jeweils das typische Impedanzspektrum direkt am Anfang einer Messreihe, d.h. unmittelbar nach der Aussaat der Zellen. Zu diesem Zeitpunkt ist noch keine barrierebildende Zellschicht auf den Membranen vorhanden. Die mit Quadraten markierten Datensätze wurden zu späteren Zeitpunkten aufgezeichnet und es ist deutlich das charakteristische Impedanzspektrum einer barrierebildenden Zellschicht zu erkennen. Wie sich aus einem Vergleich der Figuren 5 und 6 ergibt, kann auch mittels eines ersten Behältnisses, welches vollständig aus einem elektrisch leitfähigen Material besteht, die Impedanz des Zellkultursystems bestimmt werden.

### BEZUGSZEICHENLISTE

- 1: Abdeckung
- 2: Elektroden
- 3: Membransystem (= zweites Behältnis)
- 4: Bodenplatte
- 5: Kunststoffblock
- 6: erstes Behältnis
- 7: permeable Membran
- 8: Gewinde
- 9: kultivierte Zelle

## Patentansprüche

1. Eine Vorrichtung zur Bestimmung der transzellularen Impedanz von auf einer permeablen Membran kultivierten Zellen, umfassend eine oder mehrere Messzellen, wobei jede Messzelle
a) ein erstes Behältnis (6), in das ein zweites Behältnis (3) umfassend die permeable Membran als Zellkultursubstrat eingesetzt werden kann, sowie
b) eine Abdeckung (1) für das erste Behältnis (6) umfassend eine Elektrode (2), wobei die Elektrode (2) so an der Abdeckung (1) angeordnet ist, dass, wenn das zweite Behältnis (3) in dem ersten Behältnis (6) eingesetzt ist, das zweite Behältnis (3) zumindest teilweise mit einer leitenden Flüssigkeit gefüllt ist und das erste Behältnis (6) mit der Abdeckung (1) abgedeckt wird, die leitende Flüssigkeit in dem zweiten Behältnis (3) in Kontakt mit der Elektrode (2) treten kann,
umfasst, **dadurch gekennzeichnet, dass** mindestens 50 % der dem Innenraum des ersten Behältnisses (6) zugewandten Oberflächen des ersten Behältnisses (6) aus einem elektrisch leitfähigen Material gebildet sind.

2. Die Vorrichtung nach Anspruch 1, **dadurch gekennzeichnet, dass** das erste Behältnis (6) zu mindestens 50 Gew.% aus einem Metall bzw. einer metallischen Legierung gefertigt ist.

3. Die Vorrichtung nach Anspruch 1, **dadurch gekennzeichnet, dass** mindestens 50 % der dem Innenraum des ersten Behältnisses (6) zugewandten Oberflächen des ersten Behältnisses (6) mit einem Metall bzw. einer metallischen Legierung beschichtet sind.

4. Die Vorrichtung nach Anspruch 1, **dadurch gekennzeichnet, dass** das erste Behältnis (6) zu mindestens 50 Gew.% aus einem elektrisch leitfähigen Kunststoff gefertigt ist.

5. Die Vorrichtung nach Anspruch 1, **dadurch gekennzeichnet, dass** mindestens 50 % der dem Innenraum des ersten Behältnisses (6) zugewandten Oberflächen des ersten Behältnisses (6) mit einem elektrisch leitfähige Kunststoff beschichtet sind.

6. Die Vorrichtung nach einem der vorhergehenden Ansprüche, umfassend mindestens 6 Messzellen,
- wobei die mindestens 6 ersten Behältnisse (6) auf einer gemeinsamen Bodenplatte (4) angeordnet sind,
- wobei eine gemeinsame Abdeckung (1) für die mindestens 6 ersten Behältnisse (6) vorgesehen ist,
- wobei die gemeinsame Abdeckung (1) mindestens 6 Elektroden (2) umfasst, und
- wobei die mindestens 6 Elektroden (2) so an der gemeinsamen Abdeckung (1) angeordnet sind, dass, wenn jeweils das zweite Behältnis (3) in dem jeweils ersten Behältnis (6) eingesetzt ist, das jeweils zweite Behältnis (3) zumindest teilweise mit einer leitenden Flüssigkeit gefüllt ist und die mindestens 6 ersten Behältnisse (6) mit der gemeinsamen Abdeckung (1) abgedeckt werden, die leitende Flüssigkeit in jedem der zweiten Behältnisse (3) in Kontakt mit einer Elektrode (2) treten kann.

7. Die Vorrichtung nach Anspruch 6, wobei es sich bei den ersten Behältnissen (6) um Behältnisse aus Stahl handelt.

8. Die Vorrichtung nach Anspruch 6 oder 7, wobei die ersten Behältnisse (6) auf die gemeinsame Bodenplatte (4) aufgeschraubt werden können.

9. Die Vorrichtung nach einem der Ansprüche 6 bis 8, wobei die ersten Behältnisse (6) einen solchen Durchmesser aufweisen, dass in diese ersten Behältnisse (6) zweite Behältnisse (3) mit einem Durchmesser der permeablen Membran in einem Bereich von 22 bis 26 mm eingesetzt werden können.

10. Die Vorrichtung nach einem der Ansprüche 6 bis 8, wobei die ersten Behältnisse (6) einen solchen Durchmesser aufweisen, dass in diese ersten Behältnisse (6) zweite Behältnisse (3) mit einem Durchmesser der permeablen Membran in einem Bereich von 10 bis 14 mm eingesetzt werden können.

11. Die Vorrichtung nach einem der Ansprüche 6 bis 8, wobei die ersten Behältnisse (6) einen solchen Durchmesser aufweisen, dass in diese ersten Behältnisse (6) zweite Behältnisse (3) mit einem Durchmesser der permeablen Membran in einem Bereich von 5,5 bis 7,5 mm eingesetzt werden können.

12. Die Vorrichtung nach einem der Ansprüche 1 bis 5, umfassend mindestens 6 Messzellen,
- wobei die mindestens 6 ersten Behältnisse (6) in Form einer Multiwellplatte vorliegen,
- wobei eine gemeinsame Abdeckung (1) für die mindestens 6 ersten Behältnisse (6) vorgesehen ist,
- wobei die gemeinsame Abdeckung (1) mindestens 6 Elektroden (2) umfasst, und
- wobei die mindestens 6 Elektroden (2) so an der gemeinsamen Abdeckung (1) angeordnet sind, dass, wenn jeweils das zweite Behältnis (3) in dem jeweils ersten Behältnis (6) eingesetzt ist, das jeweils zweite Behältnis (3) zumindest teilweise mit einer leitenden Flüssigkeit gefüllt ist und die mindestens 6 ersten Behältnisse (6) mit der gemeinsamen Abdeckung (1) abgedeckt werden, die leitende Flüssigkeit in jedem der zweiten Behältnisse (3) in Kontakt mit einer Elektrode (2) treten kann.

13. Die Vorrichtung nach Anspruch 12, wobei die Multiwellplatte eine 6-Wellplatte ist, in deren erste Behältnisse (6) zweite Behältnisse (3) mit einem Durchmesser der permeablen Membran in einem Bereich von 22 bis 26 mm eingesetzt werden können.

14. Die Vorrichtung nach Anspruch 12, wobei die Multiwellplatte eine 12-Wellplatte ist, in deren erste Behältnisse (6) zweite Behältnisse (3) mit einem Durchmesser der permeablen Membran in einem Bereich von 10 bis 14 mm eingesetzt werden können.

15. Die Vorrichtung nach Anspruch 12, wobei die Multiwellplatte eine 24-Wellplatte ist, in deren erste Behältnisse (6) zweite Behältnisse (3) mit einem Durchmesser der permeablen Membran in einem Bereich von 5,5 bis 7,5 mm eingesetzt werden können.

16. Ein Verfahren zur Bestimmung der transzellularen Impedanz von auf einer permeablen Membran kultivierten Zellen, umfassend die Verfahrensschritte
i) Bereitstellung einer Vorrichtung nach einem der Ansprüche 1 bis 15;
ii) Einsetzten mindestens eines zweiten Behältnisses (3) umfassend eine permeable Membran als Zellkultursubstrat, auf der Zellen kultiviert sind, in mindestens eines der ersten Behältnisse (6);
iii) Befüllen des zweiten und des ersten Behältnisses (3,6) mit einer elektrisch leitfähigen Flüssigkeit, wobei die Flüssigkeit im ersten Behältnis (6) mindestens bis zu einer Füllhöhe, die bis zur permeablen Membran reicht, mit der Flüssigkeit befüllt wird;
iv) Abdecken des mindestens einen ersten Behältnisses (6) mit der Abdeckung (1);
v) Bestimmung der Impedanz der Zellschicht.

17. Die Verwendung einer Vorrichtung nach einem der Ansprüche 1 bis 15 zur Bestimmung der transzellularen Impedanz von auf einer permeablen Membran kultivierten Zellen.

## Claims

1. A device for the determination of the transcellular impedance of cells cultivated on a permeable membrane, comprising one or more measurement cells, wherein each measurement cell comprises
a) a first container (6) into which a second container (3) comprising the permeable membrane as a cell culture substrate can be inserted, and
b) a cover (1) for the first container (6) comprising an electrode (2), wherein the electrode (2) is arranged on the cover (1) such that when the second container (3) is inserted in the first container (6), the second container (3) is at least partly filled with a conducting liquid and the first container (6) is covered with the cover (1), the conducting liquid in the second container (3) can enter into contact with the electrode (2),
**characterized in that** at least 50 % of the surfaces of the first container (6) facing the interior of the first container (6) are formed from an electrically conductive material.

2. The device according to claim 1, **characterized in that** the first container (6) is produced to the extent of at least 50 wt.% from a metal or a metallic alloy.

3. The device according to claim 1, **characterized in that** at least 50 % of the surfaces of the first container (6) facing the interior of the first container (6) are coated with a metal or a metallic alloy.

4. The device according to claim 1, **characterized in that** the first container (6) is produced to the extent of at least 50 wt.% from an electrically conductive plastic.

5. The device according to claim 1, **characterized in that** at least 50 % of the surfaces of the first container (6) facing the interior of the first container (6) are coated with an electrically conductive plastic.

6. The device according to one of the preceding claims, comprising at least 6 measurement cells,
- wherein the at least 6 first containers (6) are arranged on a common base plate (4),
- wherein a common cover (1) is provided for the at least 6 first containers (6),
- wherein the common cover (1) comprises at least 6 electrodes (2), and
- wherein the at least 6 electrodes (2) are arranged on the common cover (1) such that when the particular second container (3) is inserted in the particular first container (6), the particular second container (3) is filled at least partly with a conducting liquid and the at least 6 first containers (6) are covered with the common cover (1), the conducting liquid in each of the second containers (3) can enter into contact with an electrode (2).

7. The device according to claim 6, wherein the first containers (6) are containers of steel.

8. The device according to claim 6 or 7, wherein the first containers (6) can be screwed on to the common base plate (4).

9. The device according to one of claims 6 to 8, wherein the first containers (6) have a diameter such that second containers (3) having a diameter of the permeable membrane in a range of from 22 to 26 mm can be inserted into these first containers (6).

10. The device according to one of claims 6 to 8, wherein the first containers (6) have a diameter such that second containers (3) having a diameter of the permeable membrane in a range of from 10 to 14 mm can be inserted into these first containers (6).

11. The device according to one of claims 6 to 8, wherein the first containers (6) have a diameter such that second containers (3) having a diameter of the permeable membrane in a range of from 5.5 to 7.5 mm can be inserted into these first containers (6).

12. The device according to one of claims 1 to 5, comprising at least 6 measurement cells,
- wherein the at least 6 first containers (6) are present in the form of a multi-well plate,
- wherein a common cover (1) is provided for the at least 6 first containers (6),
- wherein the common cover (1) comprises at least 6 electrodes (2), and
- wherein the at least 6 electrodes (2) are arranged on the common cover (1) such that when the particular second container (3) is inserted in the particular first container (6), the particular second container (3) is filled at least partly with a conducting liquid and the at least 6 first containers (6) are covered with the common cover (1), the conducting liquid in each of the second containers (3) can enter into contact with an electrode (2).

13. The device according claim 12, wherein the multi-well plate is a 6-well plate into the first containers (6) of which second containers (3) having a diameter of the permeable membrane in a range of from 22 to 26 mm can be inserted.

14. The device according claim 12, wherein the multi-well plate is a 12-well plate into the first containers (6) of which second containers (3) having a diameter of the permeable membrane in a range of from 10 to 14 mm can be inserted.

15. The device according claim 12, wherein the multi-well plate is a 24-well plate into the first containers (6) of which second containers (3) having a diameter of the permeable membrane in a range of from 5.5 to 7.5 mm can be inserted.

16. A method for the determination of the transcellular impedance of cells cultivated on a permeable membrane, comprising the method steps
i) provision of a device according to one of claims 1 to 15;
ii) insertion of at least one second container (3) comprising a permeable membrane as a cell culture substrate on which cells are cultivated into at least one of the first containers (6);
iii) filling of the second and the first container (3, 6) with an electrically conductive liquid, wherein the liquid in the first container (6) is filled with the liquid at least up to a filling level which extends to the permeable membrane;
iv) covering of the at least one first container (6) with the cover (1);
v) determination of the impedance of the cell layer.

17. The use of a device according to one of claims 1 to 15 for the determination of the transcellular impedance of cells cultivated on a permeable membrane.

## Revendications

1. Dispositif de détermination de l'impédance transcellulaire de cellules cultivées sur une membrane perméable, comprenant une ou plusieurs cellules de mesure, chaque cellule de mesure comprenant :
a) un premier récipient (6) dans lequel peut être mis un deuxième récipient (3) comprenant la membrane perméable à titre de substrat de culture cellulaire, et
b) un couvercle (1) pour le premier récipient (6), comprenant une électrode (2), l'électrode (2) étant disposée sur le couvercle (1) de sorte que, quand le deuxième récipient (3) est mis dans le premier récipient (6), le deuxième récipient (3) est au moins partiellement rempli d'un liquide conducteur et le premier récipient (6) est recouvert du couvercle (1), le liquide conducteur dans le deuxième récipient (3) peut entrer en contact avec l'électrode (2),
**caractérisé en ce qu'**au moins 50 % des surfaces du premier récipient (6) qui font face à l'espace interne du premier récipient (6) sont constituées d'un matériau électriquement conducteur.

2. Dispositif selon la revendication 1, **caractérisé en ce que** le premier récipient (6) est fabriqué à au moins 50 % en poids d'un métal ou d'un alliage métallique.

3. Dispositif selon la revendication 1, **caractérisé en ce qu'**au moins 50 % des surfaces du premier récipient (6) qui font face à l'espace interne du premier récipient (6) sont revêtues d'un métal ou d'un alliage métallique.

4. Dispositif selon la revendication 1, **caractérisé en ce que** le premier récipient (6) est fabriqué à au moins 50 % en poids d'une matière plastique électriquement conductrice.

5. Dispositif selon la revendication 1, **caractérisé en ce qu'**au moins 50 % des surfaces du premier récipient (6) qui font face à l'espace interne du premier récipient (6) sont revêtues d'une matière plastique électriquement conductrice.

6. Dispositif selon l'une des revendications précédentes, comprenant au moins 6 cellules de mesure,
- où les au moins 6 premiers récipients (6) sont disposés sur une plaque de fonds commune (4),
- où un couvercle commun (1) est prévu pour les au moins 6 premiers récipients (6),
- où le couvercle commun (1) comprend au moins 6 électrodes (2), et
- où les au moins 6 électrodes (2) sont disposées sur le couvercle commun (1) de sorte que, quand le deuxième récipient (3) respectif est mis dans le premier récipient (6) respectif, le deuxième récipient (3) respectif est au moins partiellement rempli d'un liquide conducteur et les au moins 6 premiers récipients (6) sont recouverts du couvercle commun (1), le liquide conducteur dans chacun des deuxièmes récipients (3) peut entrer en contact avec une électrode (2).

7. Dispositif selon la revendication 6, où les premiers récipients (6) sont des récipients en acier.

8. Dispositif selon la revendication 6 ou 7, où les premiers récipients (6) peuvent être vissés sur la plaque de fonds commune (4).

9. Dispositif selon l'une des revendications 6 à 8, où les premiers récipients présentent un tel diamètre que des deuxièmes récipients (3) ayant un diamètre de membrane perméable situé dans une plage allant de 22 à 26 mm peuvent être mis dans ces premiers récipients (6).

10. Dispositif selon l'une des revendications 6 à 8, où les premiers récipients (6) présentent un tel diamètre que des deuxièmes récipients (3) ayant un diamètre de membrane perméable situé dans une plage allant de 10 à 14 mm peuvent être mis dans ces premiers récipients (6).

11. Dispositif selon l'une des revendications 6 à 8, où les premiers récipients (6) présentent un tel diamètre que des deuxièmes récipients (3) ayant un diamètre de membrane perméable situé dans une plage allant de 5,5 à 7,5 mm peuvent être mis dans ces premiers récipients (6).

12. Dispositif selon l'une des revendications 1 à 5, comprenant au moins 6 cellules de mesure,
- où les au moins 6 premiers récipients (6) se présentent sous la forme d'une plaque multipuits,
- où un couvercle commun (1) est prévu pour les au moins 6 premiers récipients (6),
- où le couvercle commun (1) comprend au moins 6 électrodes (2), et
- où les au moins 6 électrodes (2) sont disposées sur le couvercle commun (1) de sorte que, quand le deuxième récipient (3) respectif est mis dans le premier récipient (6) respectif, le deuxième récipient (3) respectif est au moins partiellement rempli d'un liquide conducteur et les au moins 6 premiers récipients (6) sont recouverts du couvercle commun (1), le liquide conducteur dans chacun des deuxièmes récipients (3) peut entrer en contact avec une électrode (2).

13. Dispositif selon la revendication 12, où la plaque multipuits est une plaque à 6 puits, dans les premiers récipients (6) de laquelle peuvent être mis des deuxièmes récipients (3) ayant un diamètre de membrane perméable situé dans une plage allant de 22 à 26 mm.

14. Dispositif selon la revendication 12, où la plaque multipuits est une plaque à 12 puits, dans les premiers récipients (6) de laquelle peuvent être mis des deuxièmes récipients (3) ayant un diamètre de membrane perméable situé dans une plage allant de 10 à 14 mm.

15. Dispositif selon la revendication 12, où la plaque multipuits est une plaque à 24 puits, dans les premiers récipients (6) de laquelle peuvent être mis des deuxièmes récipients (3) ayant un diamètre de membrane perméable situé dans une plage allant de 5,5 à 7,5 mm.

16. Procédé de détermination de l'impédance transcellulaire de cellules cultivées sur une membrane perméable, comprenant les étapes de procédé consistant à :
i) mettre à disposition un dispositif selon l'une des revendications 1 à 15 ;
ii) mettre au moins un deuxième récipient (3) comprenant une membrane perméable à titre de substrat de culture cellulaire, sur laquelle des cellules sont cultivées, dans au moins un des premiers récipients (6) ;
iii) remplir les deuxièmes et les premiers récipients (3, 6) d'un liquide électriquement conducteur, où le liquide est rempli dans le premier récipient (6) au moins jusqu'à une hauteur de remplissage qui atteint la membrane perméable ;
iv) recouvrir le au moins un premier récipient (6) du couvercle (1) ;
v) déterminer l'impédance de la couche cellulaire.

17. Utilisation d'un dispositif selon l'une des revendications 1 à 15 pour la détermination de l'impédance transcellulaire de cellules cultivées sur une membrane perméable.
